# EUROPÄISCHE PATENTSCHRIFT

(11) **EP 1 184 046 B1**
(45) Veröffentlichungstag und Bekanntmachung des Hinweises auf die Patenterteilung: **12.11.2003**
(21) Anmeldenummer: 01120380.9
(22) Anmeldetag: 25.08.2001
(51) Int. Cl.: A61M 1/16, A61M 1/30

(54) **Blutbehandlungseinrichtung und Wegwerfeinheit für eine Blutbehandlungseinrichtung**
Blood treatment device and disposable for a blood treatment device
Appareil de traitement du sang et accessoire jetable pour un appareil de traitement du sang

(30) Priorität: 29.08.2000 DE 10042324
(43) Veröffentlichungstag der Anmeldung: 06.03.2002
(73) Patentinhaber: Fresenius Medical Care Deutschland GmbH, 61352 Bad Homburg v.d.H. (DE)
(72) Erfinder: Scheunert, Peter, 61381 Friedrichsdorf (DE); Lauer, Martin, 66606 St. Wendel (DE); Weis, Manfred, Dr., 66606 St. Wendel (DE); Beden, Josef, 55252 Mainz-Kastel (DE); Herlotz, Martin, 63150 Heusenstamm (DE); Manke, Joachim, Dr., 35792 Löhnberg (DE); Hahmann, Uwe, 75233 Tiefenbronn (DE)
(74) Vertreter: Luderschmidt, Schüler & Partner GbR

(56) Entgegenhaltungen:
- WO-A-84/02473
- DE-A- 3 328 744
- US-A- 4 486 189

## Beschreibung

Die Erfindung betrifft eine Blutbehandlungseinrichtung, insbesondere eine Dialysevorrichtung, die über eine Blutbehandlungseinheit, insbesondere einen Dialysator verfügt. Darüber hinaus bezieht sich die Erfindung auf ein Disposable für eine derartige Blutbehandlungseinrichtung.

Es sind verschiedene Dialyseverfahren bekannt. Bei der Zweinadel-Dialyse wird Blut über eine Nadel von einem Patienten abgezogen und über eine andere Nadel dem Patienten wieder zurückgeführt. Dabei wird das Blut kontinuierlich durch den Dialysator gepumpt. Die Einnadel-Dialyse erfordert nur eine einzige Nadel, um zyklisch Blut abzuziehen und wieder zuzuführen, d. h. die arterielle und venöse Blutleitung sind mit der selben Nadel verbunden.

Die Einnadel- und Zweinadel-Dialysevorrichtungen machen im allgemeinen von okkludierenden Schlauchpumpen Gebrauch. Es sind aber auch Dialysevorrichtungen bekannt, bei denen zum Pumpen des Bluts volumetrische Pumpen eingesetzt werden.

Die DE 39 11 587 A1 beschreibt eine Einnadel-Dialysevorrichtung, bei der das Blut in einer ersten Phase mit einer Nadel über einen arteriellen Zweig abgezogen und in einer zweiten Phase über einen venösen Zweig mit der gleichen Nadel wieder zurückgeführt wird. Um einen über den Dialysator kontinuierlichen Blutfluß auch bei der Einnadel-Dialyse erzielen zu können, sind in den arteriellen und venösen Zweig Ausgleichskammern geschaltet. Die Einnadel-Dialyse ist für den Patienten weniger belastend, aber die Steuerung des Blutflusses über die gemeinsame Nadel ist mit einem höheren Aufwand verbunden, als es bei der Zweinadel-Dialyse der Fall ist.

Aus der WO84/02473 und der WO98/22165 sind Blutbehandlungseinrichtungen bekannt, bei denen verschiedene Komponenten, die eine Funktionseinheit bilden, in einer Kassette zusammengefaßt sind. Diese Modulbauweise hat den Vorteil, daß sich die mit dem Blut in Berührung kommenden Teile des Systems leicht austauschen lassen. Nachteilig ist jedoch, daß der Flußplan durch die Bauweise des Moduls festgelegt ist. Sollte das Modul beispielsweise für den Zweinadel-Betrieb ausgelegt sein, ist ein Einnadel-Betrieb nicht möglich oder umgekehrt.

Der Erfindung liegt die Aufgabe zugrunde, eine Blutbehandlungseinrichtung zu schaffen, die sowohl den Einnadel- als auch Zweinadel-Betrieb erlaubt, ohne daß an dem Flußplan der Einrichtung größere Veränderungen vorzunehmen sind. Eine weitere Aufgabe der Erfindung ist, eine Wegwerfeinheit für eine derartige Blutbehandlungseinrichtung zur Verfügung zu stellen.

Die Lösung dieser Aufgabe erfolgt erfindungsgemäß mit den Merkmalen des Patentanspruchs 1 bzw. 13.

Unter einer Blutbehandlungseinrichtung werden sämtliche Einrichtungen verstanden, die über einen extrakorporalen Kreislauf mit einer Einheit zur Behandlung von Blut verfügen. Hierzu zählen insbesondere Einrichtungen zur Hämodialyse, Hämofiltration und Hämodiafiltration, aber beispielsweise auch Zellseparatoren.

Die Blutbehandlungseinrichtung weist zum Fördern von Blut zwei volumetrische Pumpen, vorzugsweise Membranpumpen auf, wobei die erste Pumpe in den ersten Leitungszweig und die zweite Pumpe in den zweiten Leitungszweig einer sich verzweigenden Zuführleitung geschaltet sind, über die das Blut von dem Patienten abgezogen und der Blutbehandlungseinheit, beispielsweise dem Dialysator zugeführt wird. Über eine Abführleitung wird das Blut aus der Blutbehandlungseinheit wieder zurückgeführt.

Für den Zweinadel-Betrieb werden sowohl an der Zuführ- als auch der Rückführleitung eine Nadel angeschlossen. Die beiden Pumpen arbeiten im Gegentakt. Während die eine Pumpe Blut ansaugt, fördert die andere Pumpe Blut in die Blutbehandlungseinheit, so daß das Blut kontinuierlich durch die Blutbehandlungseinheit strömt.

Den Einnadel-Betrieb ohne größere Veränderungen am Flußplan macht eine Verbindungsleitung zur Herstellung einer Flüssigkeitsverbindung zwischen dem Auslaß der Blutbehandlungseinheit und einer der beiden Pumpen möglich. Die Zuführ- und Rückführleitung werden beim Einnadel-Betrieb mit einer gemeinsamen Nadel verbunden. In einer ersten Phase saugen beide Pumpen über die geöffnete Flüssigkeitsverbindung Blut an, wobei das Blut durch die erste Pumpe und die Blutbehandlungseinheit zu der zweiten Pumpe fließt. Das Blut wird in dieser Phase nicht über die Rückführleitung zurückgeführt. In einer darauf folgenden zweiten Phase bleibt die Flüssigkeitsverbindung geöffnet, wobei beide Pumpen Blut fördern, das über die Rückführleitung zurückgeführt wird.

In einer alternativen Ausführungsform kann die gemeinsame Nadel nur an der Zuführleitung angeschlossen werden, wobei das Blut bei dieser Ausführungsform nur über die Zuführleitung abgezogen bzw. zurückgeführt wird.

Die mit dem Blut in Berührung kommenden Teile der Blutbehandlungseinrichtung sind vorzugsweise als Disposable ausgebildet, sie können aber auch Bestandteil der Blutbehandlungseinrichtung sein.

Die Wegwerfeinheit für die Blutbehandlungseinrichtung weist eine Zuführleitung mit zwei parallelen Leitungszweigen und eine Rückführleitung und in den ersten und zweiten Leitungszweig geschaltete Pumpkammern sowie eine Verbindungsleitung auf. Die Wegwerfeinheit kann aber noch weitere Komponenten, beispielsweise Hauptkammern etc. umfassen. Auch die Blutbehandlungseinheit kann Bestandteil der Wegwerfeinheit sein.

Die Wegwerfeinheit kann als Schlauchset ausgebildet sein. Es ist aber auch möglich, sämtliche Teile der Wegwerfeinheit in einer Funktionseinheit beispielsweise in Form eines Folienteils unterzubringen.

Die Blutbehandlungseinheit verfügt vorzugsweise über eine aus dem Stand der Technik bekannte Aufnahmeeinheit, in die die Wegwerfeinheit eingelegt wird. Die Pumpen und Sperrorgane zur Steuerung des Blutflusses sind dann sowohl Bestandteil der Wegwerfeinheit als auch der Aufnahmeinheit. Wegwerfeinheit und Aufnahmeeinheiten mit derartigen Pumpen und Sperrorganen sind beispielsweise in der DE 198 14 695 A1 bzw. W0 99/17019 beschrieben, auf die ausdrücklich Bezug genommen wird.

Die Vorteile der Erfindung kommen insbesondere dann zum Tragen, wenn die mit dem Blut in Berührung kommenden Teile als Wegwerfeinheit ausgebildet sind, das in die maschinenseitige Aufnahmeeinheit eingelegt wird. Zur Realisierung des Einnadel-Betriebs brauchen an der Wegwerfeinheit und der Aufnahmeeinheit nur geringfügige Veränderungen vorgenommen zu werden. Hierzu ist nur eine kurze, verschließbare Stichleitung erforderlich, die möglichst kurz sein sollte, so daß das Totvolumen klein ist.

Im folgenden werden mehrere Ausführungsbeispiele der Erfindung unter Bezugnahme auf die Zeichnungen näher erläutert.

Es zeigen:
- Fig. 1: die wesentlichen Komponenten einer Blutbehandlungseinrichtung für den Einnadel-Betrieb in vereinfachter Darstellung,
- Fig. 2: in vereinfachter Darstellung den eine Pumpkammer des Disposables aufnehmenden Teil der Aufnahmeeinheit der Blutbehandlungseinrichtung von Fig. 1,
- Fig. 3: eine alternative Ausführungsform der Blutbehandlungseinrichtung für den Einnadel-Betrieb und
- Fig. 4: eine Blutbehandlungseinrichtung für den Zweinadel-Betrieb.

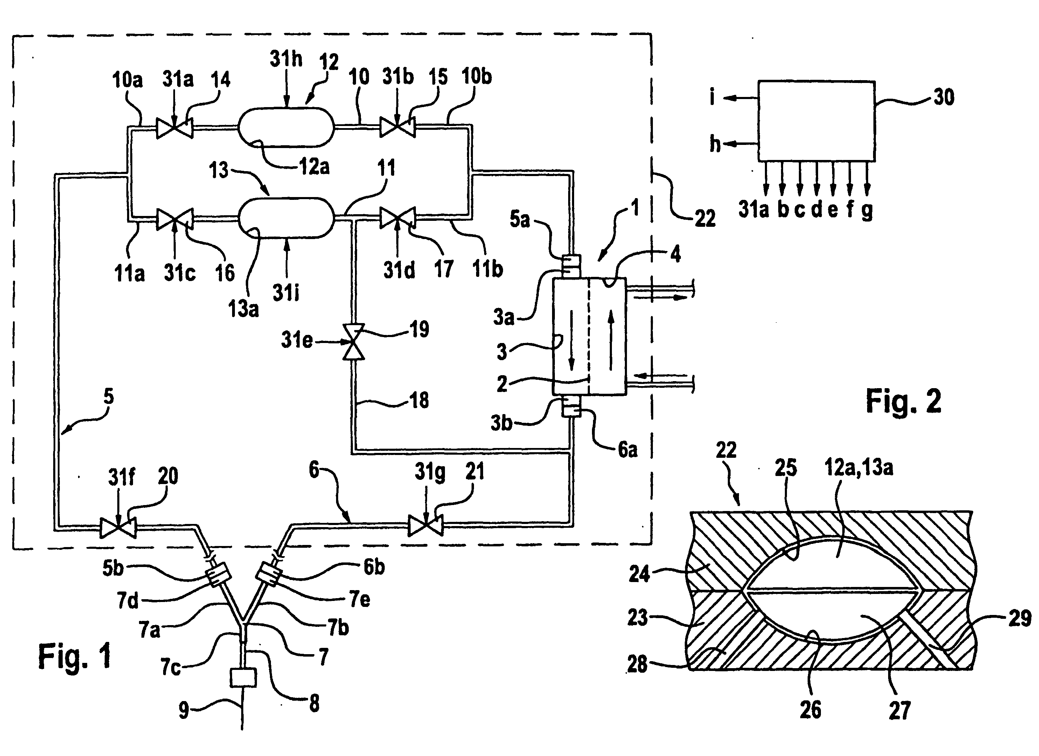

Figur 1 zeigt die wesentlichen Komponenten der Blutbehandlungseinrichtung, die eine Blutbehandlungseinheit 1 aufweist. Die Blutbehandlungseinheit 1 kann beispielsweise ein Dialysator sein, der durch eine semipermeable Membran 2 in eine Blutkammer 3 und eine Dialysierflüssigkeitskammer 4 unterteilt ist. Während der Dialysebehandlung wird die Blutkammer 3 von Blut und die Dialysierflüssigkeitskammer 4 von Dialysierflüssigkeit durchströmt. Die Flußrichtungen sind in Figur 1 durch Pfeile gekennzeichnet.

Das Blut des Patienten wird der Blutkammer 3 des Dialysators 1 über eine Zuführleitung 5 zugeführt und aus der Blutkammer über eine Rückführleitung 6 wieder zurückgeführt. Die Zufuhr- und Rückführleitung 5, 6 weisen an ihren Enden jeweils Konnektoren 5a, 5b bzw. 6a, 6b auf. Der Anschluß der Zuführ- und Rückführleitung am Ein- und Auslaß 3a, 3b der Blutkammer erfolgt mittels der Konnektoren 5a, 6a.

Für die Einnadel-Dialyse werden die freien Enden der Zufuhr- und Rückführleitung 5, 6 mittels eines Y-Verbindungsstücks 7 verbunden, das zwei einzelne Leitungszweige 7a, 7b und einen gemeinsamen Leitungszweig 7c aufweist. Zum Anschluß des Y-Verbindungsstücks 7 werden die Konnektoren 5b, 6b der Zuführ- und Rückführleitung 5, mit den entsprechenden Konnektoren 7d, 7e des Verbindungsstücks verbunden. Mit dem gemeinsamen Leitungszweig des Verbindungsstücks 7 ist eine gemeinsame Blutleitung 8 verbunden, an der eine gemeinsame Nadel 9 angeschlossen ist.

Die Zuführleitung 5 weist zwei parallele Leitungszweige 10, 11 auf, wobei in dem ersten Leitungszweig 10 eine volumentrische Pumpe, vorzugsweise eine Membranpumpe 12 und in den zweiten Leitungszweig 11 eine zweite volumetrische Pumpe, vorzugsweise eine Membranpumpe 13 geschaltet ist. In dem zu der ersten Pumpe 12 führenden Leitungsabschnitt 10a des ersten Leitungszweiges 10 ist ein erstes Sperrorgan 14, in dem von der ersten Pumpe abgehenden Leitungsabschnitt 10b des ersten Leitungszweiges 10 ein zweites Sperrorgan 15, in dem zu der zweiten Pumpe 13 führenden Abschnitt 11a des zweiten Leitungszweigs 11 ein drittes Sperrorgan 16 und in dem von der zweiten Pumpe abgehenden Leitungsabschnitt 11b des zweiten Leitungszweigs 11 ein viertes Sperrorgan 17 angeordnet.

Zur Herstellung einer Flüssigkeitsverbindung zwischen dem Auslaß der Blutkammer 3 und der zweiten Pumpe 13 ist eine Verbindungsleitung 18 vorgesehen, die von der Rückführleitung 6 abzweigt und an dem zweiten Leitungsabschnitt 11b des zweiten Leitungszweigs 11 zwischen der zweiten Pumpe 13 und dem vierten Sperrorgan 17 angeschlossen ist. In der Verbindungsleitung 18 ist ein fünftes Sperrorgan 19 vorgesehen. Ein sechstes Sperrorgan 20 ist an der Zuführleitung 5 und ein siebtes Sperrorgan 21 an der Rückführleitung 6 vorgesehen. Das sechste Sperrorgan in der Zuführleitung ist bei dieser Ausführungsform aber nicht zwingend erforderlich.

Die Zuführ- und Rückführleitung 5, 6 und die Verbindungsleitung 18 sind zusammen mit den Pumpkammern 12a, 13a der Pumpen 12, 13 als Wegwerfeinheit ausgebildet, das in eine durch gestrichelte Linien nur andeutungsweise dargestellte Aufnahmeeinheit 22 der Blutbehandlungseinrichtung eingelegt wird.

Figur 2 zeigt den Teil der Aufnahmeeinheit 22, der die Pumpkammer 12a bzw. 13a der Pumpe 12 bzw. 13 aufnimmt. Die Aufnahmeeinheit 22 besteht aus einem unteren und oberen Gehäusekörper 23, 24, die jeweils eine muldenförmige Vertiefung 25, 26 aufweisen. Die untere muldenförmige Vertiefung 25 nimmt eine Druckkammer 27 auf, die über Ein- und Auslaßkanäle 28, 29 wechselweise mit einem Gas oder eine Flüssigkeit befüllt bzw. entleert wird. In die obere muldenförmige Vertiefung 26 ist die Pumpkammer 12a bzw. 13 a der ersten bzw. zweiten Pumpe 12, 13 eingelegt. Beim Befüllen dehnt sich die Druckkammer 27 aus, so daß die Pumpkammer 12a bzw. 13a zusammengedrückt wird (Förderphase). Beim Entleeren der Druckkammer expandiert die Pumpkammer (Saugbetrieb). Derartige Aufnahmeeinheiten sind beispielsweise aus der W0 99/17019 bekannt, auf die ausdrücklich Bezug genommen wird.

Die Sperrorgane sind an den Leitungen angreifende Klemmvorrichtungen, die elektromagnetisch oder pneumatisch von einer Steuereinheit 30 betätigt werden, die über Steuerleitungen 31a bis 31g mit den einzelnen Sperrorganen verbunden ist. Die Steuereinheit 30 ist femer über Steuerleitungen 31h, 31i mit den beiden Pumpen 12, 13 verbunden.

Für die Einnadel-Dialyse wird das Disposable in die Aufnahmeeinheit eingelegt, und der Dialysator 1 und das Y-Verbindungsstück 7 werden angeschlossen. Die Steuereinheit 30 steuert die Pumpen 12, 13 und die Sperrorgane wie folgt an:

Zunächst sind alle Sperrorgane geschlossen. Zu Beginn einer ersten Phase öffnet die Steuereinheit 30 das erste, zweite, fünfte und sechste Sperrorgan 14, 15, 19, 20 und schaltet die erste und zweite Pumpe 12, 13 auf Saugbetrieb. Die erste und zweite Pumpe 12, 13 saugen nun Blut des Patienten durch die Nadel 9 so lange an, bis beide Pumpkammern 12a, 13a mit einem bestimmten Flüssigkeitsvolumen gefüllt sind. Dabei strömt Blut durch die erste Pumpkammer 12a, die Blutkammer 3 und die Verbindungsleitung 18 in die zweite Pumpkammer 13a. Wenn beide Pumpkammern 12a, 13a mit dem gewünschten Volumen gefüllt sind, werden wieder sämtliche Sperrorgane geschlossen. Daran schließt sich eine zweite Phase an. Zu Beginn der zweiten Phase schaltet die Steuereinheit 30 die erste und zweite Pumpe 12, 13 auf Förderbetrieb und öffnet das zweite, fünfte und siebte Sperrorgan 15, 19, 21. Das Blut aus den Pumpenkammern 12a, 13a wird nun über die Rückführleitung 6 durch die Nadel 9 zurückgeführt. An die zweite Phase schließt sich dann wieder die erste Phase an. Während der ersten und zweiten Phase wird die Dialysierflüssigkeitskammer 3 des Dialysators 1 kontinuierlich von Blut durchstömt. Es ist nicht zwingend erforderlich, daß zwischen den beiden Phasen alle Sperrorgane geschlossen werden. Beispielsweise können das zweite und fünfte Sperrorgan 15 und 19 geöffnet bleiben.

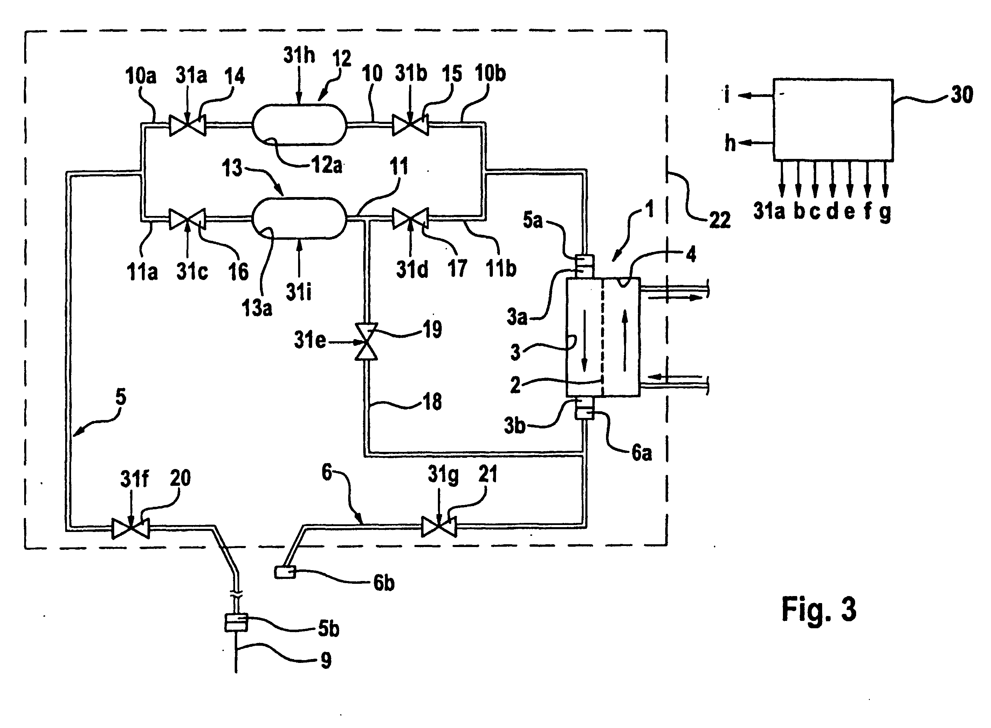

Figur 3 zeigt ein alternatives Ausführungsbeispiel der Blutbehandlungseinrichtung für die Einnadel-Dialyse. Bei diesem Ausführungsbeispiel ist die Nadel 9 direkt an dem Konnektor 5b der Zuführleitung 5 angeschlossen. Damit ist der an dem Y-Verbindungsstück 7 angeschlossene Abschnitt der Rückführleitung 6 an sich nicht erforderlich. Auf das Y-Verbindungsstück kann verzichtet werden, was den Aufbau nochmals vereinfacht. Ansonsten ist der Aufbau des Ausführungsbeispiels gemäß Figur 2 mit dem Aufbau der Ausführungsform gemäß Figur 1 identisch. Die Ansteuerung der Pumpen und Sperrorgane erfolgt bei dem Ausführungsbeispiel gemäß Figur 2 wie folgt:

In der ersten Phase schaltet die Steuereinheit 30 die erste und zweite Pumpe 12, 13 auf Saugbetrieb und öffnet das erste, zweite, fünfte und sechste Sperrorgan 14, 15, 19, 20 und schließt das dritte, vierte und siebte Sperrorgan 16, 17, 21, so daß sich die beiden Pumpkammern 12a, 13a mit Blut füllen können. Dabei strömt Blut durch die erste Kammer 12a, die Dialysierflüssigkeitskammer 3, die Verbindungsleitung 18 in die zweite Kammer 13a. In der darauffolgenden zweiten Phase schaltet die Steuereinheit 30 die Kammern 12a, 13a auf Förderbetrieb und öffnet das zweite, dritte, fünfte und sechste Sperrorgan 15, 16, 19, 20 und schließt das erste, vierte und siebte Sperrorgan 14, 17, 21, so daß das Blut aus den beiden Kammern 12a, 13a über die Zuführleitung 5 zurückgeführt wird. Dabei wird die Dialysierflüssigkeitskammer 3 von Blut durchströmt. Zwischen den beiden Phasen sind alle Sperrorgane wieder geschlossen. Dies ist aber nicht zwingend erforderlich. So kann das sechste Sperrorgan 20 auch geöffnet bleiben.

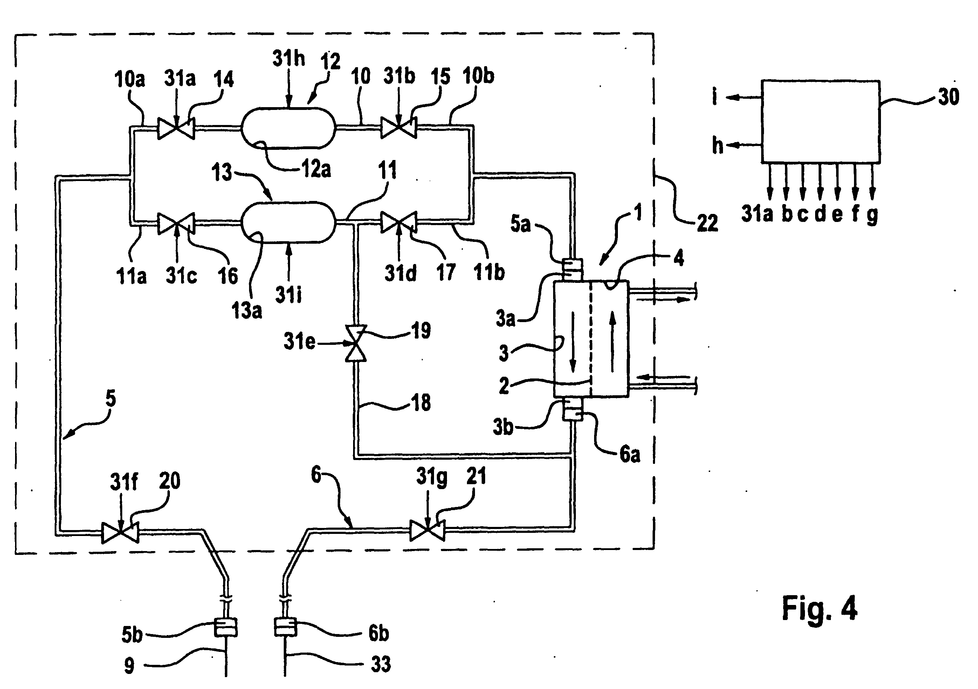

Figur 4 zeigt die Blutbehandlungseinrichtung für die Zweinadel-Dialyse. Der Aufbau entspricht den Ausführungsformen gemäß der Figuren 1 und 3. Für den Zweinadel-Betrieb wird an dem Konnektor 5b der Zuführleitung 5 eine arterielle Nadel und an dem Konnektor 6b der Rückführleitung 6 eine venöse Nadel 33 angeschlossen.

Das sechste und siebte Sperrorgan 20, 21 sind immer geöffnet, und das fünfte Sperrorgan 19 ist immer geschlossen. Während der ersten Phase schaltet die Steuereinheit 30 die erste Pumpe 12 auf Saugbetrieb und die zweite Pumpe 13 auf Förderbetrieb. Das erste und vierte Sperrorgan 14, 17 werden geöffnet und das zweite und dritte Sperrorgan 15, 16 werden geschlossen. Während die erste Pumpe 12 Blut durch die arterielle Nadel 9 über die Zuführleitung 5 ansaugt, fördert die zweite Pumpe 13 Blut in die Blutkammer 3, das über die Rückführleitung 6 durch die venöse Nadel 33 wieder zurückgeführt wird. Nach dem Füllen der ersten und Entleeren der zweiten Pumpkammer 12a,13a schaltet die Steuereinheit 30 in der zweiten Phase die erste Pumpe 12 auf Förderbetrieb und die zweite Pumpe 13 auf Saugbetrieb und öffnet das zweite und dritte Sperrorgan 15, 16 und schließt das erste und vierte Sperrorgan 14,17. Nun fördert die erste Pumpe 12 Blut in die Blutkammer 3, das durch die venöse Nadel 33 zurückgeführt wird, während die zweite Pumpe 13 Blut durch die arterielle Nadel 9 ansaugt.

Sollten sich Komplikationen mit den Gefäßzugängen eines Patienten während der Zweinadel-Dialyse ergeben, kann die erfindungsgemäße Blutbehandlungseinrichtung mit wenigen Eingriffen für die Einnadel-Dialyse umgerüstet werden. Dazu sind ggf. Veränderungen an den Nadeln 9, 33 und deren Konnektierung notwendig. Desweiteren braucht nur die Ventil- und Pumpsteuerung wie oben beschrieben verändert werden, ohne daß das in der Aufnahmeeinheit 22 eingelegte Wegwerfeinheit ausgewechselt zu werden braucht. Auf diese Weise ist eine sehr schnelle Umstellung möglich.

## Patentansprüche

1. Blutbehandlungseinrichtung mit
einer Blutbehandlungseinheit (1), die einen Einlaß (3a) und einen Auslaß (3b) aufweist,
einer zu dem Einlaß der Blutbehandlungseinheit führenden Zuführleitung (5), die zwei parallele Leitungszweige (10, 11) aufweist, und einer von dem Auslaß der Blutbehandlungseinheit abgehenden Rückführleitung (6),
einer in den ersten Leitungszweig geschalteten ersten volumetrischen Pumpe (12) und einer in den zweiten Leitungszweig geschalteten zweiten volumetrischen Pumpe (13), wobei an dem zu der ersten Pumpe führenden Leitungsabschnitt (10a) des ersten Leitungszweigs ein erstes Sperrorgan (14), an dem von der ersten Pumpe abgehenden Leitungsabschnitt (10b) des ersten Leitungszweigs ein zweites Sperrorgan (15), an dem zu der zweiten Pumpe führenden Leitungsabschnitt (11a) des zweiten Leitungszweigs ein drittes Sperrorgan (16) und an dem von der zweiten Pumpe abgehenden Leitungsabschnitt (11b) des zweiten Leitungszweigs ein viertes Sperrorgan (17) vorgesehen ist,
**gekennzeichnet durch**
eine Verbindung (18), die den Auslaß (3b) der Blutbehandlungseinheit mit einem der beiden Leitungszweige verbindet, wobei an der Verbindungsleitung ein fünftes Sperrorgan (19) vorgesehen ist.

2. Blutbehandlungseinrichtung nach Anspruch 1, **dadurch gekennzeichnet, daß** die Verbindungsleitung (18) die Rückführleitung (6) mit dem von der zweiten Pumpe (13) abgehenden Leitungsabschnitt (11b) des zweiten Leitungszweigs (11) verbindet.

3. Blutbehandlungseinrichtung nach Anspruch 1 oder 2, **dadurch gekennzeichnet, daß** die Zuführleitung (5) und die Rückführleitung (6) mit einer gemeinsamen Blutleitung (8) verbunden sind, an der eine Nadel (9) angeschlossen ist, und an der Rückführleitung ein siebtes Sperrorgan (21) vorgesehen sind, wobei eine Steuereinheit (30) zum Öffnen und Schließen der Sperrorgane sowie Umschalten der Pumpen (12, 13) vorgesehen ist, die derart ausgebildet ist,
daß in einer ersten Phase das erste, zweite und fünfte Sperrorgang (14, 15, 19) geöffnet und das dritte, vierte und siebte Sperrorgan (16, 17, 21) geschlossen sind, und in einer darauf folgenden zweiten Phase das zweite, fünfte und siebte Sperrorgan (15, 19, 21) geöffnet und das erste, dritte und vierte Sperrorgan (14, 16, 17) geschlossen sind, wobei die Pumpen zwischen der ersten und zweiten Phase von Saugbetrieb auf Förderbetrieb umgeschaltet werden.

4. Blutbehandlungseinrichtung nach Anspruch 3, **dadurch gekennzeichnet, daß** zwischen der ersten und zweiten Phase beim Umschalten der Pumpen (12, 13) das erste bis fünfte und siebte Sperrorgan geschlossen sind.

5. Blutbehandlungseinrichtung nach Anspruch 1 oder 2, **dadurch gekennzeichnet, daß** an der Zuführleitung (5) eine Nadel (9) angeschlossen ist, wobei eine Steuereinheit (30) zum Öffnen und Schließen der Sperrorgane sowie Umschalten der Pumpen (12, 13) vorgesehen ist, die derart ausgebildet ist,
daß in einer ersten Phase das erste, zweite und fünfte Sperrorgan (14, 15, 19) geöffnet und das dritte und vierte Sperrorgan (16, 17) geschlossen sind, und in einer darauf folgenden zweiten Phase das zweite, dritte und fünfte Sperrorgan (15, 16, 19) geöffnet und das erste und vierte Sperrorgan (14, 17) geschlossen sind, wobei die Pumpen zwischen der ersten und zweiten Phase von Saugbetrieb auf Förderbetrieb umgeschaltet werden.

6. Blutbehandlungseinrichtung nach Anspruch 5, **dadurch gekennzeichnet, daß** zwischen der ersten und zweiten Phase beim Umschalten der Pumpen (12, 13) das erste bis fünfte Sperrorgan geschlossen sind.

7. Blutbehandlungseinrichtung nach Anspruch 1 oder 2, **dadurch gekennzeichnet, daß** an der Zuführleitung (5) eine arterielle Nadel (9) und an der Rückführleitung eine venöse Nadel (33) angeschlossen sind, wobei eine Steuereinheit (30) zum Öffnen und Schließen der Sperrorgane sowie Umschalten der Pumpen (12, 13) vorgesehen ist, die derart ausgebildet ist,
daß in einer ersten Phase das erste und vierte Sperrorgan (14, 17) geöffnet und das zweite, dritte und fünfte Sperrorgan (15, 16, 19) geschlossen sind, und in einer darauf folgenden zweiten Phase das zweite und dritte Sperrorgan (15, 16) geöffnet und das erste, vierte und fünfte Sperrorgan (14, 17, 19) geschlossen sind, wobei die erste Pumpe (12) zwischen der ersten und zweiten Phase von Saugbetrieb auf Förderbetrieb und die zweite Pumpe (13) von Förderbetrieb auf Saugbetrieb umgeschaltet werden.

8. Blutbehandlungseinrichtung nach Anspruch 7, **dadurch gekennzeichnet, daß** zwischen der ersten und zweiten Phase beim Umschalten der Pumpen das erste bis fünfte Sperrorgan geschlossen sind.

9. Blutbehandlungseinrichtung nach einem der Ansprüche 1 bis 8, **dadurch gekennzeichnet, daß** die Sperrorgane elektromagnetische oder pneumatisch betätigbare Klemmvorrichtungen sind.

10. Blutbehandlungseinrichtung nach einem der Ansprüche 1 bis 9, **dadurch gekennzeichnet, daß** die erste und zweite Pumpe (12, 13) Membranpumpen sind.

11. Blutbehandlungseinrichtung nach einem der Ansprüche 1 bis 10, **dadurch gekennzeichnet, daß** die erste und zweite Pumpe (12, 13) jeweils eine als austauschbare Einheit ausgebildete Pumpkammer (12a, 13a) aufweisen, die in den ersten und zweiten Leitungszweig (10, 11) der Zuführleitung (5) geschaltet sind.

12. Blutbehandlungseinrichtung nach Anspruch 11, **dadurch gekennzeichnet, daß** die Zuführleitung (5), Rückführleitung (6), Verbindungsleitung (18) und die Pumpkammern (12a, 13a) der ersten und zweiten Pumpe (12, 13) als Disposable ausgebildet sind.

13. Wegwerfeinheit für eine Blutbehandlungseinrichtung nach einem der Ansprüche 1 bis 12, mit
einer Zuführleitung (5), die zwei parallele Leitungszweige (10, 11) aufweist, und einer Rückführleitung (6),
einer in den ersten Leitungszweig (10) geschalteten ersten Pumpkammer (12a) und einer in den zweiten Leitungszweig (11) geschalteten zweiten Pumpkammer (13a), und
einer Verbindungsleitung (18), die die Rückführleitung (6) mit einem der beiden Leitungszweige (10, 11) verbindet.

14. Wegwerfeinheit nach Anspruch 13, **dadurch gekennzeichnet, daß** an den freien Enden der Zuführleitung (5) und der Rückführleitung (6) Konnektoren (5b,6b) zum Anschluß von Nadeln (9 33) oder eines Y-Verbindungsstücks (7) vorgesehen sind.

## Claims

1. Blood-treatment device (1) which has one inlet (3a) and one outlet (3b),
a feed pipe (5) leading to the inlet of the blood-treatment device, which feed pipe exhibits a section containing two parallel branches (10, 11) and a return pipe (6) leading away from the outlet of the blood-treatment device,
a first volumetric pump (12) located in the first branch of the feed pipe and a second volumetric pump (13) located in the second branch of the feed pipe whereby a first flow-stopping device (14) is provided in the section (10a) of the first branch of the feed pipe leading to the first pump,
a second flow-stopping device (15) is provided in the section (10b) of the feed pipe leading away from the first pump,
a third flow-stopping device (16) is provided in the section (11a) of the second branch of the feed-pipe leading to the second pump and
a fourth flow-stopping device (17) is provided in the section (11b) of the second branch of the feed pipe leading away from the second pump
**characterised by**
a connection (18) which joins the outlet (3b) of the blood-treatment device with one of the two feed pipe branches, whereby a fifth flow-stopping device (19) is located in the connection pipe.

2. Blood-treatment device in accordance with claim 1, **characterised in that** the connection pipe (18) connects the return pipe (6) with the section (11b) leading away from the second pump (13) located in the second branch (11) of the feed-pipe.

3. Blood-treatment device in accordance with claim 1 or 2, **characterised in that** the supply feed pipe (5) and the return pipe (6) are connected by a common blood supply pipe (8) provided with a needle (9) and that a seventh flow-stopping device (21) is provided in the return pipe whereby a control unit (30) is provided with which to open and close the flow-stopping devices and to switch the pumps (12, 13) on and off, the latter being of such a design
that in a first phase the first, second and fifth flow-stopping devices (14, 15, 19) are open and the third, fourth and seventh flow-stopping devices (16, 17, 21) are closed and that in a subsequent second phase the second, fifth and seventh flow-stopping devices (15, 19, 21) are open and the first, third and fourth flow-stopping devices (14, 16, 17) are closed, whereby between the first and second phases the action of the pumps is reversed from a suction to a delivery mode.

4. Blood-treatment device in accordance with claim 3, **characterised in that** when the action of the pumps (12, 13) is reversed between the first and second phase the first to the fifth and the seventh flow-stopping devices are closed.

5. Blood-treatment device in accordance with claim 1 or 2, **characterised in that** a needle (9) is attached to the feed-pipe (5) whereby a control unit (30) is provided to open or close the flow-stopping devices and to reverse the action of the pumps (12, 13) whereby the design of this control unit is such
that in a first phase, the first, second and fifth flow-stopping devices (14, 15 19) are open and the third and fourth flow-stopping devices (16, 17) are closed - and that in a subsequent second phase the second, third and fifth flow-stopping devices (15, 16, 19) are open while the first and fourth flow-stopping devices (14, 17) are closed, whereby between the first and second phase the action of the pumps is reversed from a suction to a delivery mode.

6. Blood-treatment device in accordance with claim 5, **characterised in that** when the mode of action of the pumps (12, 13) is reversed between the first and second phase the first to the fifth flow-stopping devices are closed.

7. Blood-treatment device in accordance with claim 1 or 2, **characterised in that** an arterial needle (9) is attached to the feed pipe (5) and a venous needle (33) is attached to the return pipe whereby a control unit (30) is provided to open and close the flow-stopping devices and to reverse the action mode of the pumps, the latter being of such a design
that in a first phase the first and fourth flow-stopping devices (14, 17) are open and the second, third and fifth flow-stopping devices (15, 16, 19) are closed and that in a subsequent second phase the second and third flow-stopping devices (15, 16) are open and the first, fourth fifth flow-stopping devices (14, 17 19) are closed, whereby between the first and second phase the first pump (12) is reversed from a suction to a delivery mode and the second pump (13) is reversed from a delivery mode to a suction mode of action.

8. Blood-treatment device in accordance with claim 7, **characterised in that** the first to the fifth flow-stopping devices are closed when the action mode of the pumps is reversed between the first and second phase.

9. Blood-treatment device in accordance with one of the claims 1 to 8, **characterised in that** the flow-stopping devices are clamps which can be activated electromagnetically or pneumatically.

10. Blood-treatment device in accordance with one of the claims 1 to 9, **characterised in that** the first and second pumps (12, 13) are membrane pumps.

11. Blood-treatment device in accordance with one of the claims 1 to 10, **characterised in that** each of the first and second pumps (12, 13) possesses a pump chamber (12a, 13a) in the form of an exchangeable unit which are located in the first and second branches (10, 11) of the feed pipe (5).

12. Blood-treatment device in accordance with claim 11, **characterised in that** the feed pipe (5), the return pipe (6), the connection pipe (18) and the pump chambers (12a, 13a) of the first and second pumps (12, 13) are designed to be of a disposable nature.

13. Throw-away unit for a blood-treatment device in accordance with one of the claims 1 to 12, with
a feed pipe (5), which exhibits a section containing two parallel branches (10, 11) and a return pipe (6),
a first pump chamber (12a) located in the first branch (10) and a second pump chamber (13a) located in the second branch (11) of the feed-pipe and
a connection pipe (18) which joins the return pipe (6) with one of the two branches (10, 11) of the feed pipe.

14. Throw-away unit in accordance with claim 13, **characterised in that** connector units (5a, 5b) are provided at the free ends of the feed pipe (5) and the return pipe (6) to receive needles (9, 33) or a Y-junction piece (7).

## Revendications

1. Dispositif de traitement du sang avec :
une unité de traitement du sang (1) qui présente une entrée (3a) et une sortie (3b),
une conduite d'admission (5) menant à l'entrée du dispositif de traitement du sang, laquelle présente deux bifurcations de conduite parallèles (10, 11) et une conduite de retour (6) partant de la sortie du dispositif de traitement du sang,
une première pompe volumétrique (12) connectée sur la première bifurcation de conduite et une seconde pompe volumétrique (13) connectée sur la seconde bifurcation de conduite, moyennant quoi il est prévu sur la section de conduite (10a) de la première bifurcation de conduite menant à la première pompe, un premier organe de blocage (14), sur la section de conduite (10b) de la première bifurcation de conduite venant de la première pompe, un deuxième organe de blocage (15), sur la section de conduite (11a) de la seconde bifurcation de conduite menant à la seconde pompe, un troisième organe de blocage (16) et sur la section de conduite (11b) de la seconde bifurcation de conduite venant de la seconde pompe, un quatrième organe de blocage (17),
**caractérisé en ce qu'**une liaison (18) relie la sortie (3b) de l'unité de traitement du sang avec une des deux bifurcations de conduite, moyennant quoi il est prévu un cinquième organe de blocage (19) sur la conduite de liaison.

2. Dispositif de traitement du sang selon la revendication 1, **caractérisé en ce que** la conduite de liaison (18) relie la conduite de retour (6) avec la section de conduite (11b) de la seconde bifurcation de conduite (11) venant de la seconde pompe (13).

3. Dispositif de traitement du sang selon la revendication 1 ou 2, **caractérisé en ce que** la conduite d'admission (5) et la conduite de retour (6) sont connectées à une conduite de sang commune (8), à laquelle est raccordée une aiguille (9) et **en ce qu'**il est prévu un septième organe de blocage (21) sur la conduite de retour, moyennant quoi il est prévu une unité de commande (30) pour l'ouverture et la fermeture des organes de blocage ainsi que pour la commutation des pompes (12, 13), laquelle est formée de telle sorte que, durant une première phase, les premier, deuxième et cinquième organes de blocage (14, 15, 19) sont ouverts et les troisième, quatrième et septième organes de blocage (16, 17, 21) sont fermés et dans une seconde phase suivant celle-ci, les deuxième, cinquième et septième organes de blocage (15, 19, 21) sont ouverts et les premier, troisième et quatrième organes de blocage (14, 16, 17) sont fermés, moyennant quoi les pompes entre la première et la seconde phase passent du régime d'aspiration au régime de transport.

4. Dispositif de traitement du sang selon la revendication 3, **caractérisé en ce que**, entre la première et la seconde phase, lors de la commutation des pompes (12, 13), les organes de blocage allant du premier au cinquième et le septième organe de blocage sont fermés.

5. Dispositif de traitement du sang selon la revendication 1 ou 2, **caractérisé en ce qu'**une aiguille (9) est connectée à la conduite d'admission (5), moyennant quoi il est prévu une unité de commande (30) pour l'ouverture et la fermeture des organes de blocage ainsi que pour la commutation des pompes (12, 13), laquelle est formée de telle sorte que, durant une première phase, les premier, deuxième et cinquième organes de blocage (14, 15, 19) sont ouverts et les troisième et quatrième organes de blocage (16, 17) sont fermés et dans une seconde phase suivant celle-ci, les deuxième, troisième et cinquième organes de blocage (15, 16 19) sont ouverts et les premier et quatrième organes de blocage (14, 17) sont fermés, moyennant quoi les pompes entre la première et la seconde phase passent du régime d'aspiration au régime de transport.

6. Dispositif de traitement du sang selon la revendication 5, **caractérisé en ce que**, entre la première et la seconde phase, lors de la commutation des pompes (12, 13) les organes de blocage allant du premier au cinquième sont fermés.

7. Dispositif de traitement du sang selon la revendication 1 ou 2, **caractérisé en ce qu'**une aiguille artérielle (9) est connectée à la conduite de retour (5) et **en ce qu'**une aiguille veineuse (33) est connectée à la conduite de retour, moyennant quoi il est prévu une unité de commande (30) pour l'ouverture et la fermeture des organes de blocage ainsi que pour la commutation des pompes (12, 13), laquelle est formée de telle sorte que durant une première phase, les premier et quatrième organes de blocage (14, 17) sont ouverts et les deuxième, troisième et cinquième organes de blocage (15, 16, 19) sont fermés et **en ce que** dans une seconde phase suivant celle-ci, les deuxième et troisième organes de blocage (15, 16) sont ouverts et les premier, quatrième et cinquième organes de blocage (14, 17, 19) sont fermés, moyennant quoi la première pompe (12) entre la première et la seconde phase passe du régime d'aspiration au régime de transport et la seconde pompe (13) passe du régime de transport au régime d'aspiration.

8. Dispositif de traitement du sang selon la revendication 7, **caractérisé en ce que**, entre la première et la seconde phase, lors de la commutation des pompes, les organes de blocage allant du premier au cinquième sont fermés.

9. Dispositif de traitement du sang selon l'une des revendications 1 à 8, **caractérisé en ce que** les organes de blocage sont des dispositifs de serrage actionnés de manière électromagnétique ou pneumatique.

10. Dispositif de traitement du sang selon l'une des revendications 1 à 9, **caractérisé en ce que** la première pompe et la seconde pompe (12, 13) sont des pompes à membrane.

11. Dispositif de traitement du sang selon l'une des revendications 1 à 10, **caractérisé en ce que** la première pompe et la seconde pompe (12, 13) comportent chacune une chambre de pompage (12a, 13a) formée par une unité échangeable à laquelle sont connectées les première et seconde bifurcations (10, 11) de la conduite de retour (5).

12. Dispositif de traitement du sang selon la revendication 11, **caractérisé en ce que** la conduite d'admission (5), la conduite de retour (6), la conduite de liaison (18) et les chambres de pompage (12a, 13a) des première et seconde pompes (12, 13) sont formées en tant que matériaux à usage unique.

13. Unité à usage unique pour un dispositif de traitement du sang selon l'une des revendications 1 à 12, avec
une conduite d'admission (5), qui présente deux bifurcations parallèles (10, 11) et une conduite de retour (6),
une première chambre de pompe (12a) connectée à la première bifurcation de conduite (10) et une seconde chambre de pompe (13a) connectée à la seconde bifurcation de conduite (11), et
une conduite de liaison (18) qui relie la conduite de retour (6) avec l'une des deux bifurcations de conduite (10, 11).

14. Unité à usage unique selon la revendication 13, **caractérisée en ce qu'**il est prévu aux extrémités libres de la conduite d'admission (5) et de la conduite de retour (6) des connecteurs (5b, 6b) pour la connexion des aiguilles (9, 33) ou d'un élément de liaison en Y (7).
